# EUROPEAN PATENT APPLICATION

(11) **EP 3 647 410 A1**
(43) Date of publication of application: **06.05.2020**
(21) Application number: 17915398.6
(22) Date of filing: 13.12.2017
(51) Int. Cl.: C12N 5/071

(54) **METHOD FOR DIRECTLY REPROGRAMMING CELLS IN URINE INTO KERATINOCYTE STEM CELLS AND METHOD FOR PREPARING COMPOSITION FOR PROMOTING SKIN REGENERATION BY USING REPROGRAMMED KERATINOCYTE STEM CELLS**

(30) Priority: 30.06.2017 KR 20170083535
(71) Applicant: Stemlab Inc., Seongbuk-gu Seoul 02841 (KR)
(72) Inventor: JUN, Eun Kyoung, Seoul 02798 (KR); ZHENG, Jie, Seoul 02841 (KR); KANG, Phil Jun, Seoul 03112 (KR); SON, Da-Ryeon, Gimhae-si Gyeongsangnam-do 50949 (KR); JANG, Ji-Hoon, Jeonju-si Jeollabuk-do 55086 (KR); HONG, Won Jun, Seoul 02477 (KR); PARK, Jung-Hyun, Seoul 05315 (KR); PARK, Gyu Man, Seongnam-si Gyeonggi-do 13441 (KR); KIM, In Yong, Seoul 02475 (KR)
(74) Representative: Schiweck Weinzierl Koch Patentanwälte Partnerschaft mbB
(86) International application number: PCT/KR2017/014648
(87) International publication number: WO 2019/004533

(57) **Abstract**

The present invention relates to a method for inducing reprogramming of urine cells into keratinocyte stem cells by introducing reprogramming factors Bmi1 and dNP63a, and a composition for promoting skin regeneration which includes an induced reprogrammed keratinocyte stem cell conditioned medium as an active ingredient.

## Description

### CROSS-REFERENCE TO RELATED APPLICATION

This application claims priority to and the benefit of Korean Patent Application No. 2017-0083535, filed on 30 June 2017, the disclosure of which is incorporated herein by reference in its entirety.

### BACKGROUND

### 1. Field of the Invention

The present invention relates to a method for direct reprogramming of urine cells into keratinocyte stem cells and a method for preparing a composition for promoting skin regeneration using reprogrammed keratinocyte stem cells.

### 2. Discussion of Related Art

Stem cells refer to cells having unlimited potential towards self-renewal or differentiation related to specific cells and tissues required in the body. Stem cells are classified into three types: embryonic stem cells (ES cells) isolated from early embryos, embryonic germ cells (EG cells) isolated from embryonic primordial germ cells, and multipotent adult progenitor cells (MAPC cells) isolated from adult tissues.

Since stem cells have the potential to be differentiated into cells with specialized functions, they have been the subject of research for restoring the functions of various organs as cell therapeutic agents, and recently, their application has been expanding to the fields of plastic surgery and aesthetics.

The role of adult stem cells in vivo may be roughly summarized in two ways. First, stem cells themselves are differentiated into tissues and cells in our bodies and regenerate damaged tissues and cells, and second, stem cells continuously secrete growth factors and proteins such as cytokines during its lifespan to aid the growth and regeneration of neighboring cells.

Keratinocyte stem cells are a part of tissue comprising a single layer located deep within the skin and playing an essential role in the regeneration of skin. One of the main roles is to cause some cells to be differentiated from keratinocyte stem cells and to constitute new skin cells when the outer skin is damaged or peels off due to age, and accordingly, there is difficulty in terms of regenerating skin tissue arising from burns or deep wounds in cases that even internal keratinocyte stem cells have been destroyed. Keratinocyte stem cells are a type of adult stem cells that require invasive medical procedures for separation, and the amount of cells obtained through this procedure may be too small to be used as a therapeutic agent.

Direct reprogramming is a technique which allows conversion of somatic cells into other types of cells as desired. Since direct reprogramming does not go through a pluripotent stem cell state unlike reprogramming according to Japanese Professor Yamanaka, it can save time and exhibit a high level of efficiency, while solving the risk related to tumorigenesis and the incurrence of cost of further differentiation of pluripotent stem cells.

The development of therapeutic agents using stem cell cultures (conditioned medium) with anti-inflammatory and wound healing effects may suggest a new bioindustry model that is more successful and more promising than the development of therapeutic agents using stem cells themselves.

Keratinocyte stem cells not only have a role of simply replacing cells, but also have a role of secreting various growth factors and proteins such as cytokines which play an important role in skin regeneration. Accordingly, in the development of skin regeneration therapeutic agents, the use of keratinocyte stem cells which can have specialized use in skin regeneration represents a very logical and scientifically approach.

Thus, the present inventors have discovered a method for inducing urine cells to become reprogrammed keratinocyte stem cells having characteristics similar to those of keratinocyte stem cells as a result of introducing reprogramming factors Bmi1 and dNP63a, evaluated skin regeneration efficacy by producing a conditioned medium using reprogrammed keratinocyte stem cells, and selected main factors therefor, thereby completing the present invention.

### SUMMARY OF THE INVENTION

An objective of the present invention is to provide a composition for direct reprogramming of urine cells into keratinocyte stem cells.

Another objective of the present invention is to provide a method for direct reprogramming of urine cells into keratinocyte stem cells.

Still another objective of the present invention is to provide a method for preparing a conditioned medium using reprogrammed keratinocyte stem cells from urine cells.

Yet another objective of the present invention is to provide a composition for skin regeneration and skin wound healing, including a conditioned medium prepared using reprogrammed keratinocyte stem cells from urine cells as an active ingredient.

The present invention provides a composition for direct reprogramming urine cells into keratinocyte stem cells, including: (i) a Bmi1 protein or a nucleic acid encoding the Bmi1 protein; and (ii) a dNP63a protein or a nucleic acid encoding a dNP63a protein.

As used herein, the term "reprogrammed keratinocyte stem cells" refers to skin stem cells obtained by direct reprogramming of somatic cells and these stem cells have potential to differentiated into cells such as keratinocytes, In the specification of the present invention, reprogrammed keratinocyte stem cells are also described as induced keratinocyte stem cells.

As used herein, the term "urine cells (UCs)" is known as somatic cells that can be readily and repeatedly obtained from urine at any time without any inconvenience and pain.

The present inventors found that the already differentiated cells, urine cells, can be reprogrammed into keratinocyte stem cells with differentiation capacity by overexpressing of reprogramming factors Bmi1 and dNP63a. The term "reprogramming factors Bmi1 and dNP63a" started from reprogramming which is a concept introduced by Yamanaka in 2006. All tissues of an adult are gradually differentiated from an undifferentiated state while going through a normal development process, and thus changed into cells whose respective functions are specialized. Among them, fertilized cells are totipotent, and can be divided into an inner cell mass and outer cells when the fertilized cells become blastocysts as the subsequent development step proceeds, and in this case, inner cell mass cells can give rise to embryonic somatic cells and germ cells, which is called pluripotency. The embryonic stem cells exhibit a pluripotent-specific gene expression pattern, and representative examples thereof include Oct4, Sox2, Nanog, Lin28, and the like. Reprogramming can be called a technology that induces such specific gene expression in somatic cells to restore properties similar to those of undifferentiated cells such as embryonic stem cells and adult stem cells. Bmi1 and dNP63a are two of the various factors that have been used in a series of studies, and used by the present inventor in a technique that overexpression of a gene by introducing the gene into cells using a viral vector.

The Bmi1 and dNP63a used for the composition of the present invention include all of the Bmi1 and dNP63a derived from animals such as human, horses, sheep, pigs, goats, camels, antelopes, and dogs, and are preferably human Bmi1 and dNP63a. Further, the Bmi1 and dNP63a protein of the present invention used for direct reprogramming of urine cells include not only proteins having a wild type amino acid sequence thereof, but also variants of Bmi1 and dNP63a proteins.

The variants of Bmi1 and dNP63a proteins refer to proteins, having at least one amino acid residue which differs from the native amino acid sequences of Bmi1 and dNP63a by deletion, insertion, non-conservative or conservative substitution, or a combination thereof. The variant may be a functional equivalent that shows the same biological activity as a native protein, or may be a variant in which physical and chemical properties of a protein are modified as necessary. The variant is a variant with the structural stability which is increased under certain physical or chemical conditions, or physiological activity which is increased.

A nucleic acid encoding Bmi1 and dNP63a proteins is a nucleic acid comprising coding sequence of wild type Bmi1 and dNP63a proteins or Bmi1 and dNP63a proteins in variant form as described above, may be modified by substitution, deletion, and insertion of one or more bases, or a combination thereof, and may be naturally isolated or prepared using a chemical synthesis method.

The nucleic acids encoding Bmi1 and dNP63a proteins may be singlestranded or double-stranded, and may be a DNA molecule (genome, cDNA) or an RNA molecule.

In one preferred aspect, the composition for reprogramming of urine cells into keratinocyte stem cells in the present invention may comprise vectors constructed with coding sequence of Bmi1 and dNP63a that can express Bmi1 and dNP63 a proteins.

As used herein, the term "vector" refers to an expression vector capable of expressing a target protein in suitable host cells, and a gene construct including an essential regulatory element that is operably linked to express a gene insert.

The term "operably linked" refers to a functional linkage between a nucleic acid expression control sequence and a nucleic acid sequence encoding a target protein that carry out a general function. An operable linkage with a recombinant vector may be prepared using enzymes based site-specific DNA cleavage and ligation gene recombination technique. The vector of the present invention may include a signal sequence or leader sequence for membrane targeting or secretion in addition to expression regulatory elements such as a promoter, an operator, an initiation codon, a termination codon, a polyadenylation signal, and an enhancer, and may be variously prepared according to the purpose thereof. The promoter of the vector may be constitutive or inducible. Further, the expression vector includes a selectable marker for selecting a host cell containing a vector, and a replication origin in the case of being a replicable expression vector. The vector may be self-replicated or integrated into a host DNA.

The vector includes plasmid vector, cosmid vector, viral vector, and the like. Preferably, the vector is a viral vector. The viral vector includes a retrovirus vector, for example, human immunodeficiency virus (HIV), murine leukemia virus (MLV), avian sarcoma/leukosis (ASLV), spleen necrosis virus (SNV), rous sarcoma virus (RSV), mouse mammary tumor virus (MMTV), and the like, adenovirus vector, an adeno-associated virus, a herpes simplex virus, and the like, but is not limited thereto. A Moloney murine leukemia virus (MMLV)-based virus vector, pMXs vector, was used in the present invention.

Coding sequence of Bmi1 and dNP63a proteins may be delivered to cells by a publicly-known method, for example, transduction of a vector driven naked DNA into cells (Wolff et al. Science,1990: Wolff et al. J Cell Sci. 103:1249-59, 1992), or may be delivered to cells using a liposome, a cationic polymer, and the like. The liposome is a phospholipid membrane prepared by mixing a cationic phospholipid such as DOTMA or DOTAP for gene delivery, and a nucleic acid-liposome complex is formed by mixing a cationic liposome and an anionic nucleic acid in a predetermined ratio.

As another preferred aspect, the composition for direct reprogramming of urine cells into keratinocyte stem cells in the present invention may comprise viruses expressing Bmi1 and dNP63a.

As used herein, the term "virus" refers to a virus expressing Bmi1 and dNP63a constructed by transforming and infecting a viral vector comprising a coding sequence of Bmi1 and dNP63a with packaging cells.

The virus which may be used for preparation of a virus expressing Bmi1 and dNP63a proteins of the present invention may comprise a retrovirus, an adenovirus, an adeno-associated virus, a herpes simplex virus, and the like, but is not limited thereto. Preferably, the virus is a retrovirus.

In a specific example of the present invention, urine cells were infected by transforming packaging cells 293gpg cells producing a high titer virus capable of infecting a broad range of mammalian host cells with vectors (pMXs-Bmil and pMXs-dNP63a) constructed by inserting conding sequence of Bmi1 and dNP63a into a pMXs vector to prepare a virus expressing Bmi1 and dNP63a proteins.

The present invention provides a method for direct reprogramming of urine cells into kerotinocyte stem cells, the method comprise: transduction of coding sequence of Bmi1 and dNP63a into urine cells.

More specifically, the method may comprise:
(a) Isolating urine cells from urine and culturing the urine cells;
(b) Transducing cording sequence of Bmi1 and dNP63a into the isolated and cultured urine cells;
(c) Culturing the transduced urine cells under keratinocyte stem cell culture conditions; and
(d) Selecting a reprogrammed keratinocyte stem cell line having characteristics similar to keratinocyte stem cells from the urine cells which have been cultured.

In step (a), urine cells may be cultured in a medium containing fetal bovine serum (FBS), a basic fibroblast growth factor (bFGF), and an epithelial growth factor (EGF).

As used herein, the term "culture medium" refers to a medium capable of supporting the growth and survival of cells *in vitro,* and comprises all the typical media used in the art suitable for the induction and culture of urine cells and reprogrammed keratinocyte stem cells. According to the type of cell, a culture medium and culture conditions may be selected. The culture medium used for the culture is preferably a cell culture minimum medium (CCMM), and generally comprises a carbon source, a nitrogen source, and trace element components. Examples of such a cell culture minimum medium comprise a Dulbecco's Modified Eagle's Medium (DMEM), a Minimal Essential Medium (MEM), a Basal Medium Eagle (BME), RPMI1640, F-10, F-12, an α Minimal Essential Medium (αMEM), a Glasgow's Minimal Essential Medium (GMEM), Iscove's Modified Dulbecco's Medium (IMEM), and the like, but are not limited thereto. Further, the culture medium may comprise an antibiotic such as penicillin, streptomycin, or gentamicin.

In a specific example of the present invention, the urine cells may be obtained by culturing the cells isolated from urine in a basal medium containing FBS, bFGF, and EGF, and specifically, the urine cells may be obtained by adding bFGF and EGF to high glucose DMEM and a renal epithelial cell growth medium (REGM) including FBS and culturing the cells isolated from urine in the culture medium. More specifically in the present invention, the high glucose DMEM and REGM may additionally comprise L-glutamine and penicillin-streptomycin.

In step (c), reprogrammed keratinocyte stem cells may be induced by culturing urine cells into which nucleic acids encoding Bmi1 and dNP63a proteins are introduced in a basal medium containing FBS and EGF, and more specifically, reprogrammed keratinocyte stem cells may be induced by adding insulin, hydrocortisone, cholera toxin, T3, ascorbic acid, and EGF to a high glucose DMEM/F12 3 : 1 medium containing FBS, and culturing the urine cells. As a preferred example of the present invention, the high glucose DMEM/F12 3 : 1 medium may additionally comprise L-glutamine and penicillin-streptomycin.

The reprogrammed keratinocyte stem cells selected in step (d) may be cultured in a basal medium containing FBS and EGF, and specifically, the reprogrammed keratinocyte stem cells may be cultured in DMEM/F12 3 : 1 medium containing insulin, hydrocortisone, cholera toxin, T3, ascorbic acid, EGF and FBS. In a specific example of the present invention, the high glucose DMEM/F12 3 : 1 medium may additionally comprise L-glutamine, and penicillin-streptomycin.

Further, the present invention provides a method for preparing a reprogrammed keratinocyte stem cell conditioned medium or a culture solution thereof from urine cells, the method comprising:
(a) Isolating urine cells from urine and culturing the urine cells;
(b) Transducing cording sequence of Bmi1 and dNP63a into the isolated and cultured urine cells;
(c) Culturing the transduced urine cells under keratinocyte stem cell culture conditions; and
(d) Selecting a reprogrammed keratinocyte stem cell line having characteristics similar to keratinocyte stem cells from the urine cells which have been cultured; and
(e) Acquiring a conditioned medium or a culture solution thereof by culturing the selected reprogrammed keratinocyte stem cells in a serum-free medium.

The contents related to steps (a) to (d) are the same as the contents described above, and thus will be omitted.

As used herein, the "conditioned medium" refers to a medium in which when cells are cultured in a liquid suspension and reach the exponential growth phase which is the peak phase of cell division, divided cells are removed by centrifugation or filtration, and only a culture solution is extracted, and mixed with a culture substrate. The conditioned medium contained unknown growth factor which is extracted from dividing cells, and is frequently used for low-density cell plating or protoplast culture. In an example of the present invention, a culture solution obtained from the conditioned medium or medium was used.

In step (e), the serum-free medium may be a DMEM/F12 medium, and FBS containingmedium obtained by adding insulin, hydrocortisone, cholera toxin, T3, ascorbic acid, and EGF to a high glucose DMEM/F12 3 : 1 medium. As a more specific example, the high glucose DMEM/F12 3 : 1 medium may additionally comprise L-glutamine and penicillin-streptomycin. The conditioned medium may be prepared by culturing reprogrammed keratinocyte stem cells in the serum-free medium for 1 to 5 days, and more specifically, may be prepared by culturing reprogrammed keratinocyte stem cells for 3 days.

The conditioned medium prepared by the method or the culture solution thereof may be a composition comprising a material such as a growth factor derived from reprogrammed keratinocyte stem cells, and more specifically, may comprise one or more materials selected from activin A, angiogenin (ANG), coagulation factorIII, CXCL16, DPPIV, endostatin/collagenXVIII, a basic Fibroblast Growth Factor (bFGF), a granulocyte macrophage colony-stimulating factor (GM-CSF), an insulin-like growth factor-binding protein 1 (IGFBP-1), IGFBP-2, IL-1β, IL-8, CCL2/MCP-1, CCL3/MIP1α, pentraxin3 (PTX3), a platelet-derived growth factor (PDGF-AA), a placental growth factor (PIGF), Serpin B5/Maspin, Serpin E1/PAI-1, a tissue inhibitor of metalloproteinase 1 (TIMP-1), thrombospondin-1 (TSP-1), a urokinase-type plasminogen activator (uPA), and a vascular endothelial growth factor (VEGF). However, the conditioned medium or the culture solution thereof is not limited thereto. More preferably, the conditioned medium or the culture solution thereof may comprise a basic fibroblast growth factor (bFGF), a platelet-derived growth factor (PDGF-AA), and a vascular endothelial growth factor (VEGF), which are skin regeneration promoting growth factors, among the materials contained in the conditioned medium.

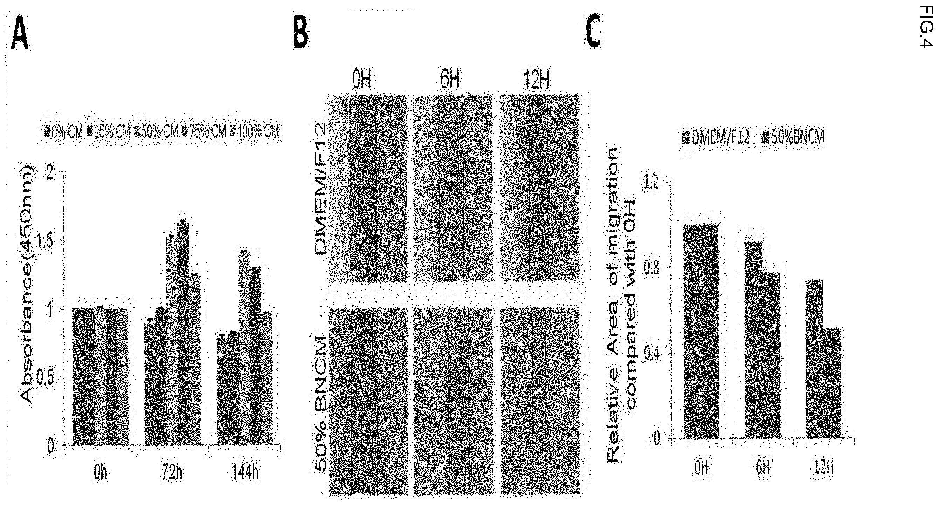

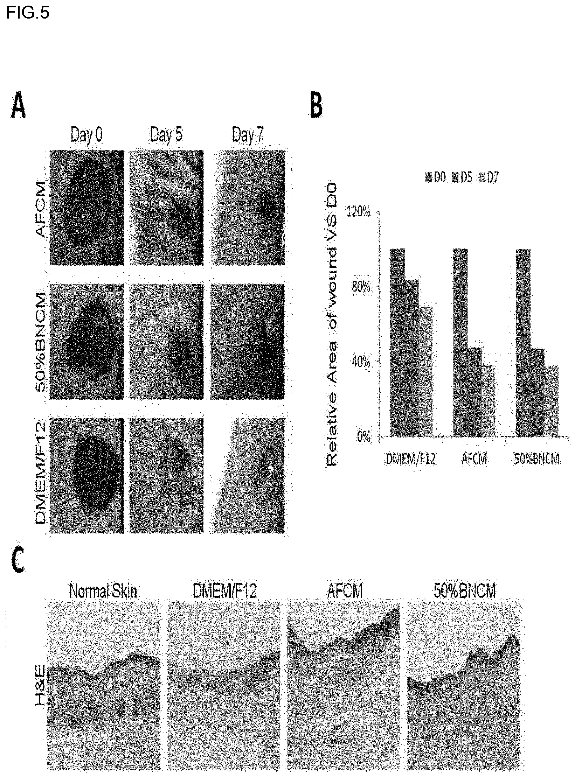

The reprogrammed keratinocyte stem cell conditioned medium prepared by the present invention or a culture solution thereof is effective for skin regeneration. In a specific example of the present invention, as a result of artificially wounding skin-derived fibroblast or mouse skin, and then treating the skin-derived fibroblast or mouse skin with the conditioned medium of the present invention, it could be confirmed that the wound healing effects are excellent because the degree of migration of fibroblast is higher (FIGs. 4B, 4C and 5).

Furthermore, the present invention provides a cosmetic composition for promoting skin regeneration, comprising the prepared conditioned medium or a culture solution thereof as an active ingredient.

The ingredient included in the cosmetic composition of the present invention comprises ingredients typically used for a cosmetic composition in addition to the reprogrammed keratinocyte stem cell conditioned medium or a culture solution thereof as an active ingredient, and comprises, for example, a typical adjuvant such as an antioxidant, a stabilizer, a solubilizer, a vitamin, a pigment, and a fragrance, and a carrier. Further, the cosmetic composition may additionally comprise a skin absorption-promoting material in order to enhance the effects thereof.

The cosmetic composition of the present invention may be prepared as any formulation typically prepared in the art, and may be formulated as, for example, a solution, a suspension, an emulsion, a paste, a gel, a cream, a lotion, a powder, a soap, a surfactant-containing cleanser, an oil, a powder foundation, an emulsion foundation, a wax foundation, a spray, or the like, but not limited thereto. More specifically, the cosmetic composition of the present invention may be prepared as a formulation such as a softening toner, a nutrient toner, a nutrient cream, a massage cream, an essence, an eye cream, a cleansing cream, a cleansing foam, a cleansing water, a pack, a spray, or a powder. When the formulation of the present invention is a paste, a cream or a gel, an animal oil, a vegetable oil, a wax, paraffin, starch, traganth, a cellulose derivative, a polyethylene glycol, silicone, bentonite, silica, talc, zinc oxide, or the like may be used as the carrier component.

When the formulation of the present invention is a powder or a spray, lactose, talc, silica, aluminum hydroxide, calcium silicate, or a polyamide powder may be used as the carrier component, and in particular, when the formulation is a spray, the formulation may additionally include a propellant such as chlorofluorohydrocarbon, propane/butane or dimethyl ether. When the formulation of the present invention is a solution or an emulsion, a solvent, a solubilizer or an emulsifier is used as the carrier component, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or fatty acid esters of sorbitan. When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, traganth, or the like may be used as the carrier component. When the formulation of the present invention is a surfactant-containing cleanser, aliphatic alcohol sulfate, aliphatic alcohol ether sulfate, sulfosuccinic acid monoester, isethionate, an imidazolinium derivative, methyltaurate, sarcosinate, fatty acid amide ether sulfate, alkylamidobetain, an aliphatic alcohol, fatty acid glyceride, fatty acid diethanolamide, a vegetable oil, a lanolin derivative, an ethoxylated glycerol fatty acid ester, or the like may be used as the carrier component.

Further, the present invention provides a pharmaceutical composition for treating skin wound, including the prepared conditioned medium or a culture solution thereof.

The pharmaceutical composition of the present invention includes a pharmaceutically acceptable carrier. A pharmaceutically acceptable carrier included in the composition of the present invention is a pharmaceutically acceptable carrier typically used in formulation, and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. The pharmaceutical composition of the present invention may additionally include a lubricant, a wetting agent, a sweetening agent, a flavoring agent, an emulsifier, a suspending agent, a preservative, and the like, in addition to the aforementioned ingredients. Suitable pharmaceutically acceptable carriers and formulations are described in detail in Remington's Pharmaceutical Sciences (19th ed., 1995).

The pharmaceutical composition of the present invention may be administered to a mammal such as a mouse, a rat, a livestock, and a human via various routes such as oral or parenteral administration, and may be administered, for example, by oral, rectal or intravenous, intramuscular, subcutaneous, intrauterine epidural, or intracerebroventricular injection. Preferably, during the parenteral administration, the pharmaceutical composition of the present invention is applied by transdermal administration, and more preferably, the pharmaceutical composition of the present invention is applied by a topical administration method.

An adequate dose of the pharmaceutical composition of the present invention may vary depending on factors, such as formulation method, administration method, age, body weight, gender or disease condition of the patient, diet, administration time, administration route, excretion rate, and response sensitivity. When the pharmaceutical composition of the present invention is an oral formulation, the pharmaceutical composition of the present invention may be administered in an amount of 0.1 to 100 mg/kg based on an adult once to several times daily, and when the pharmaceutical composition of the present invention is an external preparation, the composition may be continuously applied in an amount of 1.0 to 3.0 ml based on an adult once to five times daily for 1 month or more. However, the dosage is not intended to limit the scope of the present invention.

The pharmaceutical composition of the present invention may be prepared in the form of a unit dosage or by being contained in a multi-dose container by being formulated using a pharmaceutically acceptable carrier and/or excipient according to a method that can be readily implemented by a person with ordinary skill in the art to which the present invention pertains. In this case, the formulation may be used in any form suitable for a pharmaceutical preparation including an oral formulation such as a powder, a granule, a tablet, a capsule, a suspension, an emulsion, a syrup, or an aerosol, an external preparation such as an ointment or a cream, a suppository, a sterilized injection solution, and the like, and may additionally include a dispersant or a stabilizer.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1A illustrates the number of co-positive colonies of KRT15/ITGA6 which is a keratinocyte stem cell marker after inducing direct reprogramming of urine cells into keratinocyte stem cells using a retroviral vector system to introduce a reprogramming factor Bmi1(B)/dNP63a(N)/Klf4(K) into the urine cells at each combination (BNK, BN, BK, NK, B, N, and K);
FIG. 1B is a result of performing an RT-PCR experiment in order to confirm whether the combination of the reprogramming factors is expressed in urine cells;
FIG. 1C illustrates a result of inducing reprogrammed keratinocyte stem cells using male or female-derived urine cells;
FIG. 2A illustrates that reprogrammed keratinocyte stem cell lines BN28-2, BN28-5, and BN28-6 are established using a colony-picking method to select co-positive colonies of KRT15/ITGA6 which is a keratinocyte stem cell marker and subject the colonies to expansion culture;
FIG. 2B confirmed that the reprogrammed stem cells were keratinocyte stem cells by confirming the expression of keratinocyte stem cell markers ANGPTL2, CD200, ITGA6, KRT15, KRT14, and GJB2 in the established keratinocyte stem cell lines;
FIGS. 2C and 2D are results of confirming the stability of reprogrammed keratinocyte stem cells induced at each combination (BNK, BN, NK, and N) using Bmi1(B)/dNP63a(N)/Klf4(K);
FIG. 2E is a result of confirming the stability of reprogrammed keratinocyte stem cells induced at each combination (BNK and BN) using Bmi1(B)/dNP63a(N)/Klf4(K);
FIG. 3 is a result of confirming the potential to differentiate reprogrammed keratinocyte stem cells into keratinocytes; (A) a result of confirming keratinocyte markers at the mRNA level through qPCR experiments, (B) a result of confirming keratinocyte markers at the protein level through aimmunochemistry experiments;
FIG. 4 is a result of verifying skin regeneration efficacies according to the concentration of reprogrammed keratinocyte stem cell-derived conditioned medium in vitro; (A) observation of cell growth according to the concentration of the conditioned medium; (B) and (C) comparison of wound healing effects through observation of migration situations of fiber cells in a 50% conditioned medium and a DMEM/F12 3 : 1 basal medium;
FIG. 5 is a result of verifying skin regeneration efficacies according to the composition of reprogrammed keratinocyte stem cell-derived conditioned medium in vivo;
FIG. 6 confirmed the analysis of components of the conditioned medium derived from reprogrammed stem cells through cytokine array experiments; and
FIG. 7 is a result of measuring the amounts of skin regeneration promoting growth factors PDGF-AA, bFGF, and VEGF Protein using ELISA.

### DETAILED DESCRIPTION OF EXEMPLARY EMBODIMENTS

Exemplary embodiments of the present invention will be described in detail below with reference to the accompanying drawings. While the present invention is shown and described in connection with exemplary embodiments thereof, it will be apparent to those skilled in the art that various modifications can be made without departing from the spirit and scope of the invention.

Hereinafter, the present invention will be described in more detail with reference to Examples. However, the following Examples are provided only to more easily understand the present invention, and the present invention is not limited to the following Examples.

### Example 1: Isolation of urine cells from urine

After urine cells were centrifuged at 2,000 rpm for 10 minutes by collecting 200 ml of urine in a container, the supernatant thereof was removed. After the pellet was washed with PBS including an antibiotic and then centrifuged at 2,000 rpm for 10 minutes, the supernatant thereof was removed. The cell pellets were seeded onto a 12-well plate coated with gelatin by resuspending the cell pellets with a DMEM/F12 1 : 1 medium including an antibiotic + 10% FBS.

After 1 ml of the DMEM/F12 1 : 1 medium including an antibiotic + 10% FBS was added daily for 5 days, the medium was replaced with a medium of 5% FBS, bFGF 2.5 ng/ml, EGF 2.5 ng/ml, 44% high glucose DMEM including 0.5% L-glutamine and 0.5% penicillin-streptomycin, and a 50% renal epithelial cell growth medium (REGM). When confluence reached 95% or more, urine cells were cultured (passaging) through subculture. At Passage 3, urine cells were used to induce reprogrammed keratinocyte stem cells.

### Example 2: Selection of combination of reprogramming factors for inducing of urine-derived reprogrammed keratinocyte stem cell lines

In order to obtain reprogrammed keratinocyte stem cell lines, urine cells were induced into reprogrammed keratinocyte stem cells using a retroviral vector system to introduce reprogramming factors Bmi1 (B, NCBI ID:648, RefSeq: NM_005180.8), dNP63a(N, NCBI ID:8626,RefSeq: NM_001114980.1), and Klf4(K, NCBI ID: 9314, RefSeq: NM_001314052.1) into urine cells at each combination (BNK, BN, BK, NK, B, N, and K). The specific method is as follows:
In the urine cells isolated and cultured in Example 1, vectors (pMXs-Bmil, pMXs-dNP63a, and pMXs-Klf4) constructed by inserting nucleotide sequences encoding Bmil, dNP63a and Klf4 proteins into a pMXs vector were infected with a virus into which the reprogramming factor combination prepared using a human 293-derived retroviral packaging cell line (293GPG) was introduced, thereby introducing the reprogramming factors into urine cells.

The urine cells into which the reprogramming factors were introduced were cultured in a high glucose DMEM/F12 3 : 1 medium including 1% L-glutamine, 1% penicillin-streptomycin, 5 ug/ml insulin, 0.5 ug/ml hydrocortisone, 8.3 ng/ml cholera toxin, 1.37 ng/ml T3, 52.8 ug/ml ascorbic acid, 20 ng/ml EGF, and 2% FBS.

The cultured urine cells were each fixed with 4% paraformaldehyde for 20 minutes, and then washed three times with PBS + 0.1%BSA. Thereafter, after bloking and permeabilization (with a nuclear and cytoplasmic marker for 40 minutes, and with a surface marker for 10 minutes) were performed using 0.3% Triton X100 (PBS+1%BSA+10% donkey serum), the cells were washed three times with PBS+0.1%BSA. And then, after the cells were reacted with a primary antibody (PBS+1% BSA+10% donkey serum) at room temperature for 1 hour, and then washed three times with PBS+0.1% BSA, the cells were reacted with a secondary antibody (PBS+0.1% BSA) at room temperature for 1 hour, and then washed three times with PBS+0.1% BSA. And then, after the cells were reacted with DAPI (PBS+0.1% BSA) at room temperature for 5 minutes, the cells were washed three times with PBS+0.1%BSA, and then observed under a microscope. For the primary antibody and the secondary antibody, keratinocyte stem cell markers KRT15 and ITGA6 were used.

Further, a RT-PCR experiment was performed as follows in order to confirm whether the reprogramming factors were properly overexpressed by viral infection:
An mRNA encoding Bmil, dNP63a, Klf4 or a combination thereof for confirming the expression was isolated from cell using TRIzol. After the mRNA was prepared into a cDNA by a T3000 Thermocycler (Biometra) device using oligDT and Reverse Transcriptase II, specific sequence fragments were confirmed using primers (pBMN5: GCTTGGATACACGCCGC (SEQ ID No. 1); Bmi-Reverse: TTGCTGGTCTCCAGGTAACG (SEQ ID NO. 2); dNP63a-Reverse: ATGATGAACAGCCCAACCTC (SEQ ID No. 3); Klf4-Reverse: TGTACACCGGGTCCAATT (SEQ ID No. 4)) specific for Bmil, dNP63a or Klf4 genes to perform PCR in the T3000 Thermocycler device. GAPDH was used as a house-keeping gene.

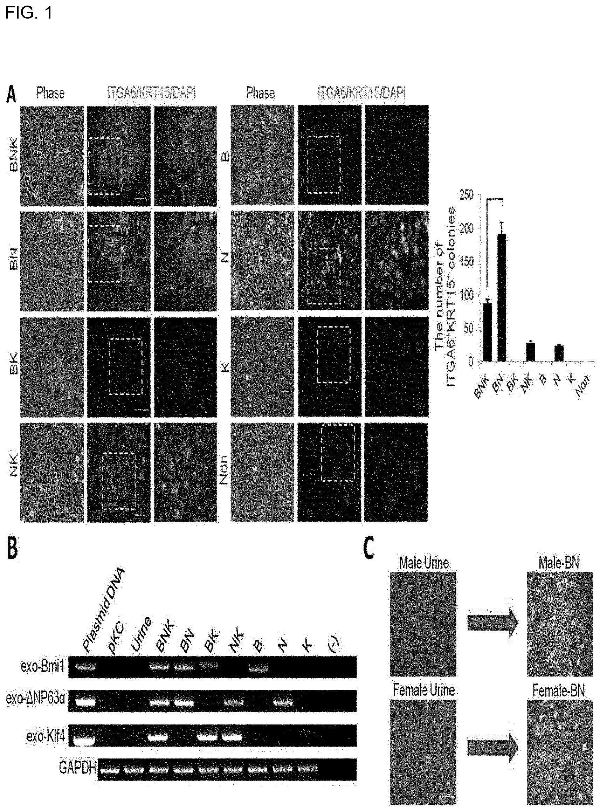

As a result, a gene combination of Bmi1/dNP63a(BN), which exhibited the highest efficiency through the number of co-positive colonies of KRT15 and ITGA6 which is a keratinocyte stem cell-specific marker, was selected as an optimal combination (FIG. 1A). In order to confirm whether reprogramming factors were properly overexpressed in cells, an RT-PCR experiment was performed, and it could be confirmed that exogenous Bmi1/dNP63a/Klf4 genes of each combination were expressed at the mRNA level (FIG. 1B). Regardless of gender, the gene combination Bmi1/dNP63a (BN) could successfully induce urine cells into dediffrentiated keratinocyte stem cells (FIG. 1C).

### Example 3: Establishment of urine-derived reprogrammed keratinocyte stem cell lines into which Bmi1 and dNP63a are introduced

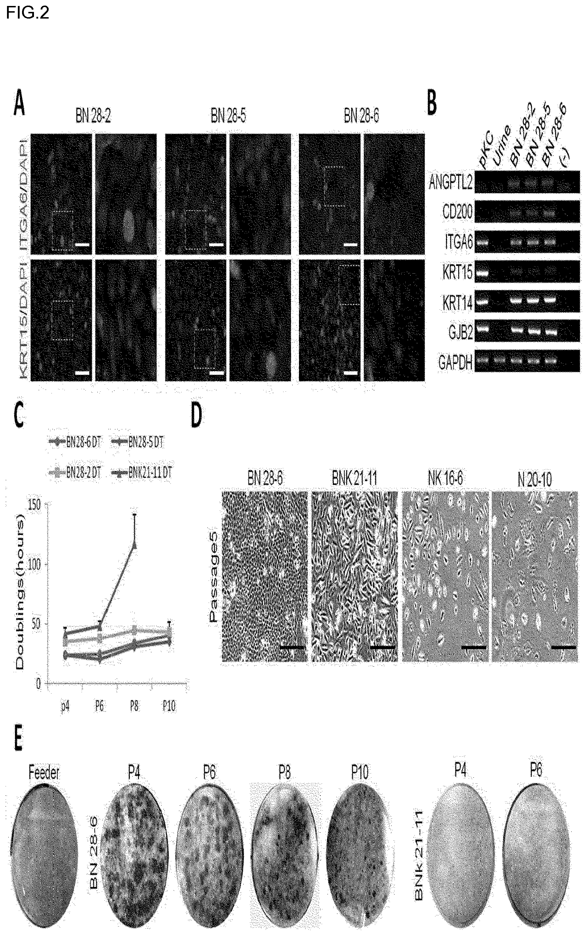

After colonies co-positive for KRT15/ITGA6 which is a keratinocyte stem cell-specific marker were selected from colonies similar to keratinocyte stem cells among cells induced in Example 2 using a colony-picking method, reprogrammed keratinocyte stem cells BN28-2, BN28-5, and BN28-6 were established by expansion culturing the colonies in a high glucose DMEM/F12 3 : 1 medium including 1% L-glutamine, 1% penicillin-streptomycin, 5 ug/ml insulin, 0.5 ug/ml hydrocortisone, 8.3 ng/ml cholera toxin, 1.37 ng/ml T3, 52.8 ug/ml ascorbic acid, 20 ng/ml EGF, and 10% FBS (FIG 2A).

With respect to the established cell lines, the expression of the keratinocyte stem cell markers ANGPTL2, CD200, ITGA6, KRT15, KRT14, and GJB2 at the mRNA level was confirmed using primers (ANGPTL2-Forward: TACATGGCACAACGGCAAGCA (SEQ ID No. 5); ANGPTL2-Reverse: TTGGAGTGGGCACAGGCGTTAT (SEQ ID No. 6); CD200-Forward: AATGGGACCACGTCTGTTAC (SEQ ID No. 7); CD200-Reverse: GCGGAACTGAAAACCAATAGC (SEQ ID No. 8); ITGA6-Forward: TGCACGCGGATCGAGTTTGA (SEQ ID No. 9); ITGA6-Reverse: AACACCGCCCAAAGATGTCTCG (SEQ ID No. 10); KRT15-Forward: TGCAGTCCCAGCTCAGCATG (SEQ ID No. 11); KRT15-Reverse: ATGCCAATGCCAGCCATCTT (SEQ ID No. 12); KRT14-Forward: TGTGGAAGCCGACATCAATG(SEQ ID No. 13); KRT14-Reverse: CTCTCAGGGCATTCATCTCC (SEQ ID No. 14); GJB2-Forward: AGGAGATCAAAACCCAGAAGG (SEQ ID No. 15); and GJB2-Reverse: AAGACGTACATGAAGGCGG (SEQ ID No. 16);) specific for the markers to perform an RT-PCR experiment.

Further, a long-term culture capacity was confirmed by calculating the doubling-time of each cell line at each subculture (passage), and after each cell line was seeded on a 3T3J2 feeder at 2500 cell/well (6 well plate), and then cultured for 7 days, the formation of colonies was confirmed by performing crystal violet staining as follows:
after the colonies were fixed with 10% formaldehyde or 4% paraformaldehyde for 20 minutes, and then washed twice with distilled water, the colonies were reacted for 20 minutes using a 0.05% crystal violet solution, washed approximately ten times with water, and dried overnight, and then the colonies were observed under a microscope or with the naked eye.

As a result, the expression of keratinocyte stem cell specific markers ANGPTL2, CD200, ITGA6, KRT15, KRT14, and GJB2 could be confirmed, and thus, it was confirmed that the colonies were reprogrammed into keratinocyte stem cells (FIG. 2B). In addition, during the continuous subculture, the reprogrammed keratinocyte stem cells established through the BN combination could maintain the growth rate and cell state for a longer period of time than the other combinations (BNK, NK, or N) (FIGs. 2C and 2D). In order to confirm stem cell properties of the established reprogrammed keratinocyte stem cell lines, a colony-forming experiment was performed, and it was confirmed that the reprogrammed keratinocyte stem cell lines established by the BN combination could maintain stem cell properties for a longer period of time than the BNK combination (FIG. 2E).

### Example 4: Confirmation of differentiation potential of reprogrammed keratinocyte stem cells

After the reprogrammed keratinocyte stem cell lines established in Example 3 were cultured in the medium to which 1.2 mM Ca²⁺ was added in Example 3 for 7 days, a differentiation experiment of the reprogrammed keratinocyte stem cells was performed. In order to confirm the differentiation into keratinocytes, by using a qPCR experiment, the expression of the markers was confirmed using primers (KRT18-Forward: AGACCATGCAAAGCCTGAAC (SEQ ID No. 17); KRT18-Reverse: GCAGTCGTGTGATATTGGTGTC (SEQ ID No. 18); KRT8-Forward: CTCAGAGATCAACCGGAACA (SEQ ID No. 19); KRT8-Reverse: TTCATCAGCTCCTGGTACTCAC (SEQ ID No. 20); KRT1-Forward: TCTGGCTCTGCTGGGATCATCA (SEQ ID No. 21); KRT1-Reverse: TCCGACTTCCAAATCCACCACC (SEQ ID No. 22); Involucrin-Forward: CTCCTCCAGTCAATACCCATCAG (SEQ ID No. 23); Involucrin-Reverse: ACATTCTTGCTCAGGCAGTCC (SEQ ID No. 24); Filaggrin-Forward: TTCGGCAAATCCTGAAGAATC (SEQ ID No. 25); and Filaggrin-Reverse: CTTGAGCCAACTTGAATACCATC (SEQ ID No. 26);) specific for the keratinocyte markers (ITGA6, KRT14, KRT18, KRT8, KRT1, Involucrin, and Filaggrin) at the mRNA level.

Further, the keratinocyte specific marker at the protein level was confirmed using an immunochemistry experiment. In the immunochemistry experiment, the same method as in Example 2 was performed, and for the primary antibody and the secondary antibody, the keratinocyte specific markers ITGA6, KRT14, KRT18, KRT8, KRT1, Involucrin, and Filaggrin were used.

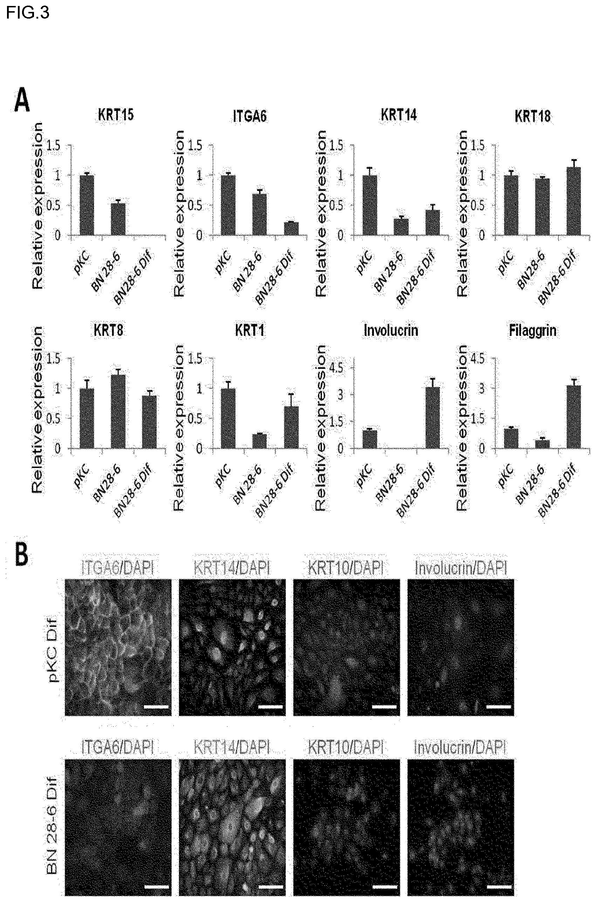

As a result, it could be confirmed that the expression of the keratinocyte specific markers KRT1, Involucrin, and Filaggrin was improved more than that of reprogrammed keratinocyte stem cells, whereas the expression of the reprogrammed keratinocyte stem cell specific marker KRT15 was reduced (FIG. 3A). In addition, through the immunochemistry experiment, the expression of the keratinocyte specific markers ITGA6, KRT14, KRT10, and Involucrin could be confirmed at the protein level (FIG. 3B).

### Example 5: Verification of production and in vivo skin regeneration efficacy of reprogrammed keratinocyte stem cell-derived conditioned medium

When confluence reached 95% or more in a 100 mm dish using the reprogrammed keratinocyte stem cell lines established in Example 3, a conditioned medium was prepared by culturing the stem cell lines in a serum-free DMEM/F12 3 : 1 medium for 3 days. After preparing the conditioned medium at various percentage concentrations, the fiber cells were treated with the conditioned medium to confirm the cell growth for 5 days by a cck-8 method of treating the reprogrammed keratinocyte stem cell lines treated with the medium with a cck-8 solution for 1 hour, and then measuring absorbance at 450 nm. As a result, when the cell lines were treated with the conditioned media at a concentration of 50% and 75%, the highest growth without any difference rate was exhibited, and the 50% reprogrammed keratinocyte stem cell-derived conditioned medium was used for the verification of skin regeneration efficacy (FIG. 4A).

In order to confirm the skin regeneration efficacy of the reprogrammed keratinocyte stem cell-derived conditioned medium in vitro, an in vitro scratch assay experiment was performed as follows: 7 X 10⁶ of confluenced fiber cells were seeded onto a 6 well-plate cells, and 6 hours later, scratches were formed using a 200-µL pipette tip, and then treated with the conditioned medium to observe the migration situation of fiber cells for 12 hours by a microscope. As a result, when the fiber cells were treated with the 50% reprogrammed keratinocyte stem cell-derived conditioned medium, a much better wound closure effect was confirmed than when the fiber cells were treated with a basal medium of 3: 1 mixture of DMEM/F12 (FIGs. 4B and 4C).

### Example 6: Verification of in vivo skin regeneration efficacies of reprogrammed keratinocyte stem cell-derived conditioned medium

In order to verify the in vivo skin regeneration efficacies of the reprogrammed keratinocyte stem cell-derived conditioned medium, ICR mice were used. After hairs were removed from the backs of the ICR mice, a 8-mm wound was physically created on the skin. The skin regeneration situation was observed by treating each wound with an amniotic fluid-derived mesenchymal stem cell-derived conditioned medium, a 50% reprogrammed keratinocyte stem cell-derived conditioned medium, and a basal medium of 3 : 1 mixture of DMEM/F12 daily for 7 days. According to the result, it could be confirmed that when the wound was treated with a 50% reprogrammed keratinocyte stem cell-derived conditioned medium, the wound recovery situation exhibited much better effects than when wound was treated with the basal medium of 3 : 1 mixture of DMEM/F12, and it was confirmed that the wound recovery effect exhibited a result similar to that of the amniotic fluid-derived mesenchymal stem cell-derived conditioned medium already known to exhibit a good effect of skin regeneration (FIGS. 5A and 5B). By histologically confirming the wounds with H&E staining, it could be confirmed that when the wounds were treated with a 50% reprogrammed keratinocyte stem cell-derived conditioned medium, cicatricial tissues were formed similarly to when the wounds were treated with the amniotic fluid-derived mesenchymal stem cell-derived conditioned medium (FIG. 5C).

### Example 7: Analysis of ingredients of reprogrammed keratinocyte stem cell-derived conditioned medium

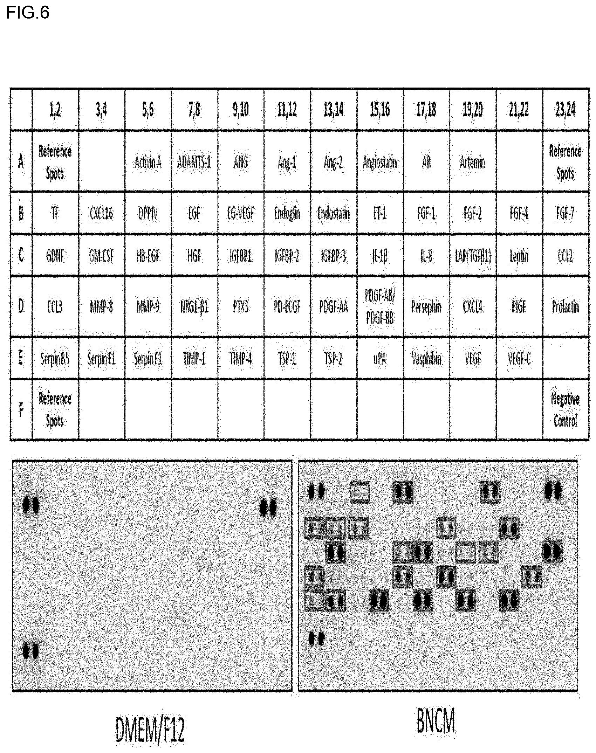

In order to analyze the ingredients of the reprogrammed keratinocyte stem cell-derived conditioned medium, a cytokine array was performed using an R&D ARY007 cytokine array kit. In the reprogrammed keratinocyte stem cell-derived conditioned medium, various growth factors and cytokines such as activin A, angiogenin (ANG), coagulation factorIII, CXCL16, DPPIV, endostatin/collagenXVIII, a basic Fibroblast Growth Factor (bFGF), a granulocyte macrophage colony-stimulating factor (GM-CSF), an insulin-like growth factor-binding protein 1 (IGFBP-1), IGFBP-2, IL-1β, IL-8, CCL2/MCP-1, CCL3/MIP1α, pentraxin3 (PTX3), a platelet-derived growth factor (PDGF-AA), a placental growth factor (PIGF), Serpin B5/Maspin, Serpin E1/PAI-1, a tissue inhibitor of metalloproteinase 1 (TIMP-1), thrombospondin-1 (TSP-1), a urokinase-type plasminogen activator (uPA), and a vascular endothelial growth factor (VEGF) were confirmed as components thereof (FIG. 6).

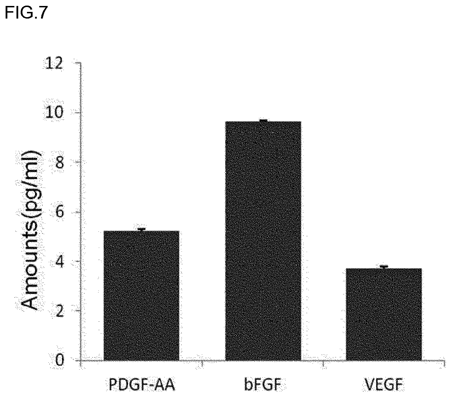

In particular, the amounts of skin regeneration promoting growth factors PDGF-AA, bFGF, and VEGF were determined by measuring the concentrations of PDGF-AA, bFGF, and VEGF using a RayBio® Human VEGF-A, PDGF-AA, bFGF ELISA kit (FIG. 7). According to the measurement, PDGF-AA, bFGF, and VEGF were present in an amount of about 5.2 pg/mL, about 9.6 pg/mL, and about 3.7 pg/mL, respectively. It could be confirmed that a skin regeneration-related protein was also included in the reprogrammed keratinocyte stem cell-derived conditioned medium.

The present invention can mass-produce personally-customized reprogrammed keratinocyte stem cells using urine cells known as somatic cells which can be easily and repeatedly obtained at any time without any inconvenience and pain. Furthermore, a conditioned medium including various human growth factors prepared using the reprogrammed keratinocyte stem cells can be prepared and can also be applied to the intractable disease field which can be expanded into the skin wound healing and skin generation fields and to a technique for producing cell therapeutic agents.

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

## Claims

1. A composition for direct reprogramming urine cells into keratinocyte stem cells, the composition comprising:
(i) a Bmi1 protein, or a coding sequence of Bmi1 protein; and
(ii) a dNP63a protein, or a coding sequence of dNP63a protein.

2. The composition of claim 1, wherein the urine cells are somatic cells derived from urine.

3. The composition of claim 1, wherein the composition comprises an expression vector into which coding sequence of Bmi1 protein and dNP63a protein are inserted.

4. The composition of claim 1, wherein the composition comprises a virus into which coding sequence of Bmi1 protein and dNP63a protein are introduced.

5. A method for direct reprogramming urine cells into keratinocyte stem cells, the method comprising:
(a) Isolating urine cells from urine and culturing the urine cells;
(b) Transducing cording sequence of Bmi1 and dNP63a into the isolated and cultured urine cells;
(c) Culturing the transduced urine cells under keratinocyte stem cell culture conditions; and
(d) Selecting a reprogrammed keratinocyte stem cell line having characteristics similar to keratinocyte stem cells from the urine cells which have been cultured.

6. The method of claim 5, wherein the transduction of coding sequence of Bmi1 and dNP63a in step (b) by infection of the urine cells with retrovirusexpression of Bmi1 and dNP63a.

7. The method of claim 5, wherein the culture conditions in step (c) comprise culturing the urine cells in a high glucose DMEM/F12 3 : 1 medium containing fetal bovine serum (FBS), insulin, hydrocortisone, cholera toxin, T3, ascorbic acid, an epidermal growth factor (EGF), L-glutamine, and penicillin-streptomycin.

8. A method for preparing a reprogrammed keratinocyte stem cell conditioned medium or a culture solution thereof from urine cells, the method comprising:
(a) Isolating urine cells from urine and culturing the urine cells;
(b) Transducing cording sequence of Bmi1 and dNP63a into the isolated and cultured urine cells;
(c) Culturing the transduced urine cells under keratinocyte stem cell culture conditions; and
(d) Selecting a reprogrammed keratinocyte stem cell line having characteristics similar to keratinocyte stem cells from the urine cells which have been cultured; and
(e) Acquiring a conditioned medium or a culture solution thereof by culturing the selected reprogrammed keratinocyte stem cells in a serum-free medium.

9. The method of claim 8, wherein the transduction of coding sequence of Bmi1 and dNP63a in step (b) comprises infecting the urine cells with retrovirus comprising coding sequence of Bmi1 and dNP63.

10. The method of claim 8, wherein the culture conditions in step (c) comprise culturing the urine cells in a high glucose DMEM/F12 3 : 1 medium including fetal bovine serum (FBS), insulin, hydrocortisone, cholera toxin, T3, ascorbic acid, an epidermal growth factor (EGF), L-glutamine, and penicillin-streptomycin.

11. The method of claim 8, wherein the conditioned medium comprises VEGF, PDGF-AA, and bFGF produced from reprogrammed keratinocyte stem cells.

12. A cosmetic composition for promoting skin regeneration, the cosmetic composition comprising as an active ingredient the reprogrammed keratinocyte stem cell conditioned medium or culture solution thereof prepared by the method of any one of claims 8 to 11.

13. A pharmaceutical composition for treating skin wounds, the pharmaceutical composition comprising as an active ingredient the reprogrammed keratinocyte stem cell conditioned medium or culture solution thereof prepared by the method of any one of claims 8 to 11.
